# EUROPEAN PATENT SPECIFICATION

(11) **EP 2 533 749 B1**
(45) Date of publication and mention of the grant of the patent: **01.09.2021**
(21) Application number: 10703066.0
(22) Date of filing: 09.02.2010
(51) Int. Cl.: A61K 6/816, A61K 6/818, A61K 6/822, C04B 35/486

(54) **A PROCESS FOR IMPROVING THE STABILITY OF YTTRIUM STABILISED ZIRCONIA FOR DENTAL RESTORATIONS**
VERFAHREN ZUR VERBESSERUNG DER STABILITÄT VON YTTRIUMSTABILISIERTEM ZIRKONOXID FÜR ZAHNERSÄTZE
PROCÉDÉ POUR L'AMÉLIORATION DE LA STABILITÉ DE ZIRCONE STABILISÉE PAR DE L'YTTRIUM POUR DES RESTAURATIONS DENTAIRES

(43) Date of publication of application: 19.12.2012
(73) Proprietor: Vita Zahnfabrik H. Rauter GmbH & Co. KG, 79713 Bad Säckingen (DE)
(72) Inventor: THOLEY, Michael, 79713 Bad Säckingen (DE); THIEL, Norbert, 79713 Bad Säckingen (DE)
(74) Representative: dompatent von Kreisler Selting Werner - Partnerschaft von Patent- und Rechtsanwälten mbB
(86) International application number: PCT/EP2010/051547
(87) International publication number: WO 2011/098115

(56) References cited:
- EP-A2- 0 364 281
- WO-A1-2004/009503
- WO-A2-2008/106958
- CA-A1- 1 251 305
- US-A1- 2002 031 675
- US-A1- 2004 232 576
- Brenntag Canada Inc.: "Propellant A31/isopentane blends", , 13 January 2009 (2009-01-13), pages 1-8, Retrieved from the Internet: URL:https://www.edocr.com/v/vznqn0mv/globa ldocuments/PROPELLANT-A31-ISOPENTANE-BLEND S [retrieved on 2018-02-01]
- DRUMMOND J L: "IN VITRO AGING OF YTTRIA-STABILIZED ZIRCONIA", JOURNAL OF THE AMERICAN CERAMIC SOCIETY, BLACKWELL PUBLISHING, MALDEN, MA, US, vol. 72, no. 4, 1 January 1989 (1989-01-01), page 675/676, XP001196975, ISSN: 0002-7820, DOI: 10.1111/J.1151-2916.1989.TB06194.X

## Description

The present invention pertains to a process for improving the stability of yttrium stabilised zirconia for dental restorations, and the use of a substantially water-free aerosol for the manufacturing of a veneered dental restoration.

### Background of the invention

Zirconia holds an exclusive place amongst dental restorative materials compared with other oxide ceramics, such as alumina, because of its excellent mechanical properties as a consequence of transformation toughening that was identified in the mid-1970s. Pure zirconia can exist in three different crystal structures depending on temperature. At room temperature up to 1170°C, the symmetry is monoclinic, the crystals are tetragonal between 1170 and 2370°C and cubic above 2370°C up to the melting point. The transformation from tetragonal (t) to monoclinic (m) during a cooling process is accompanied by a volume increase (approximately 4%) and shear distortion, sufficient to cause catastrophic failure. Alloying pure zirconia with stabilizing oxides such as Y₂O₃ allows the preservation of the meta-stable tetragonal structure at room temperature and therefore a potential control of a stress-induced t-m transformation, which can efficiently enhance resistance to crack extension leading to higher toughness compared to alumina.

As a consequence of the metastability of tetragonal zirconia, stress-generating surface treatments, such as grinding or sandblasting, are able to trigger the t→m transformation with the associated volume increase leading to the formation of surface compressive stresses, thereby increasing the flexural strength. However such alloying also alters the phase integrity of the material and increases the susceptibility to aging. The low temperature degradation (LTD) of zirconia is a well-documented phenomenon, dependent upon the presence of moisture and modest heat. The consequences of this aging process are multiple and include surface degradation with grain pullout and micro-cracking as well as strength degradation. Although LTD has been shown to be associated with a series of orthopedic hip prostheses failures in 2001 and despite a well established definition of the conditions under which L TD occurs, there is currently no clear relationship between LTD and failure predictability when zirconia is used as a dental bio-ceramic.

3Y - TZP is now widely used in dentistry for the fabrication of dental restorations (see US 2004/232576 A1 and WO 2008/106958 A2), mostly processed by machining of partially-sintered blanks followed by sintering at high temperature. The mechanical properties of 3Y-TZP strongly depend on its grain size. Above a critical size, Y - TZP is less stable and more vulnerable to spontaneous t→m transformation than smaller grain sizes (<1 µm). Moreover, below a certain grain size (~ 0.2 µm), the stress-induced transformation is not possible, leading to reduced fracture toughness. Veneering of dental restorations is discussed in WO 2004/009503 A1 and EP 0364281 A2.

### Summary of the invention

One object of the invention was to provide a process avoiding the drawbacks of the prior art, in particular the generation of a transformation of the tetragonal to the monoclinic state during manufacturing of a veneered yttrium stabilized zirconia restoration.

According to the invention this is achieved by a process for improving the stability of yttrium stabilised zirconia for dental restorations comprising yttrium stabilised zirconia, the dental restorations are veneered with a dental ceramic veneering material wherein the dental restoration comprising yttrium stabilised zirconia and to be veneered is provided; the dental ceramic veneering material is applied to the dental restoration by an aerosol which contains the dental ceramic veneering material and substantially water-free propellant; and the resulting arrangement fired to a resulting veneered dental restoration. In particular, the dental ceramic veneering material is applied by the aerosol in a thin layer on a framework of the dental restoration. The layer has a thickness of from about 0.01 mm to 0.5 mm, in particular about 0.1 mm.

The method of the invention avoids destabilization of the yttrium stabilised zirconia by introduction of moisture when the veneering material is applied to the yttrium stabilised zirconia dental restoration.

In one embodiment of the invention the yttrium stabilised zirconia dental restoration comprises a composition with zirconium dioxide, yttrium oxide and optionally hafnium oxide, aluminium oxide, and silica.

In another embodiment of the invention the dental ceramic veneering material comprises the composition comprising SiO₂; Al₂O₃; K₂O; Na₂O; B₂O₃; optionally BaO; TiO₂; CeO₂; ZrO₂; CaO; SnO₂.

In yet another embodiment of the invention the dental ceramic veneering material is present in a particulate form.

The aerosol is applied by spraying-on the yttrium stabilised zirconia dental restoration. The dental ceramic veneering material is released from a container by a propellant.

Still another embodiment of the invention is the use of a substantially water-free aerosol for the manufacturing of a veneered dental restoration the aerosol comprising a propellant and a dental ceramic veneering material for spraying-on an yttrium stabilised zirconia dental restoration to be veneered with the dental ceramic veneering material.

The method of the invention avoids destabilization of the yttrium stabilised zirconia by introduction of moisture when the veneering material is applied to the yttrium stabilised zirconia dental restoration.

Every kind of yttrium stabilised zirconia dental restorations can be veneered according to the invention. In particular bridges, crowns, inlays, onlays and veneers can be manufactured.

### Detailed description of the invention

The yttrium stabilised zirconia dental restoration comprises a composition with of from about 93 to about 97 % by weight zirconium dioxide, of from about 3 to about 6 % by weight yttrium oxide, of from about 0 to about 3 % by weight hafnium oxide, of from about 0 to about 1 % by weight aluminium oxide, and of from about 0 to about 1 % by weight silicon oxide.

The yttrium stabilised zirconia dental restoration in particular comprises a composition with of from about 93 to about 95 % by weight zirconium dioxide, of from about 4,8 to about 5,5 % by weight yttrium oxide, of from about 0 to about 3 % by weight hafnium oxide, of from about 0 to about 0,4 % by weight aluminium oxide.

In another embodiment of the invention the dental ceramic veneering material comprises the composition of from about 58 to about 70 % by weight SiO₂; of from about 10 to about 18 % Al₂O₃by weight; of from about 6 to about 12 % by weight K₂O; of from about 3 to about 8 % by weight Na₂O; of from about 1 to about 6 % by weight B₂O₃; of from about 0 to about 3 % by weight BaO; of from about 0 to about 2 % by weight TiO₂; of from about 0 to about 2 % by weight CeO₂; of from about 0 to about 2 % by weight ZrO₂; of from about 0 to about 3 % by weight CaO; of from about 0 to about 2 % by weight SnO₂.

In particular, the dental ceramic veneering material comprises a composition of from about 60 to about 64 % by weight SiO₂; of from about 13 to about 15 % Al₂O₃ by weight; of from about 7 to about 10 % by weight K₂O; of from about 4 to about 6 % by weight Na₂O; of from about 3 to about 5 % by weight B₂O₃; of from about 1 to about 3 % by weight BaO; of from about 0 to about 0,5 % by weight TiO₂; of from about 0 to about 0,5 % by weight CeO₂; of from about 0 to about 1 % by weight ZrO₂; of from about 1 to about 2 % by weight CaO; of from about 0 to about 0,5 % by weight SnO₂.

In yet another embodiment of the invention the dental ceramic veneering material is present in a particulate form, in particular of a powder having in particular a particle size of from about 0.5 µm to about 150 µm, in particular a particle size of from about 0.5 µm to about 50 µm

The dental ceramic veneering material is released from a container by a propellant which is in particular selected from the group consisting of aerosols based on heptafluoro propane or propane, butane or mixtures thereof.

Still another embodiment of the invention is the use of a substantially water-free aerosol for the manufacturing of a veneered dental restoration the aerosol comprising a propellant and a dental ceramic veneering material for spraying-on an yttrium stabilised zirconia dental restoration to be veneered with the dental ceramic veneering material.

The application of the ceramic veneering material by a spraying-on method prevents or avoids the transformation from t→m of the zirconia crystal at the surface of the framework, because no aqueous liquid can attack the crystals during the first veneering step. Furthermore, the surface of the zirconia framework material becomes protected by the applied thin layer of ceramic veneering material during following veneering steps and of the saliva after the insertion. After the thin protection layer of the sprayed ceramic powder is applied and fired; the dental technician can build up by using a brush and a mixture of dental ceramic powder with liquid a veneering layer in the right shape of the dental restoration. This technique is the known technique for building a dental restoration on a framework. After the application of the powder liquid mixture the technicians has to follow the recommended firing schedule of the manufacturer of the dental ceramic powder. Normally it is needed one build up dentin firing and a glaze firing step.

After a milling process the yttria partially stabilised zirconia material has to be veneered with ceramic material to receive a natural look of the dental restoration. According to the prior art typically a powder of dental ceramic and modelling liquid or distilled water is mixed to form a slip. The ceramic material is then applied on the zirconia framework with a brush by a dental technician. The restoration is then fired to a firing end temperature above 700°-950°C with a heating temperature speed of about 30°C - 60°C per min following a preheating time and temperature.. The liquid or its steam causes during the firing process a transformation process from t→m of the yttria partially stabilised zirconia on the surface of the framework material. The t→m transformation affects a volume increase of the zirconia crystals and with this energy impact a nucleation growth process which may cause cracks in the framework material with a destabilisation of the dental restoration.

The application of the spraying-on method of the invention avoids or minimizes the transformation because no aqueous liquid can affect the framework material during the first firing process. Furthermore, the thin layer of porcelain will protect the framework of the transformation process by the application of the following veneering steps, which can be performed by a brush with a normal dental liquid powder mixture.

The invention is further described by the following examples which do not limit the scope of the invention.

The application of the ceramic veneering powder with the spraying-on method can be performed by an aerosol which contains the dental ceramic veneering material and substantially water-free propellant. The yttria stabilised zirconia framework has been produced by a milling machine and then sintered as recommended by the manufacturer of the framework material e.g. at 1380°C or at 1530°C.

From a distance of about 20 cm to the framework the spray of dental ceramic veneering powder has been applied on the outer surface of the framework by spraying a thin layer of approximately 0.1 mm.

After this step the framework with the completely covered outer surface has to be fired in a first firing veneering step at the recommended end temperature with the recommended heating speed and the recommended preheating time and temperature for the veneering ceramic. Following this firing step the thin veneered surface of the yttria stabilised zirconia material has a brilliant glossy shine. Because of the absence of a aqueous liquid medium no transformation process from t→m occurs at the surface of the yttria stabilised zirconia.

Onto this surface the veneering ceramic with the mixture of powder and liquid and with the method by a brush can be applied. The dental technician can build up the dental restoration in the way he is used. The surface of the yttria stabilised zirconia will be protected from the aqueous liquid influence in the following veneering steps and no transformation process of the zirconia crystals will occur.

## Claims

1. A process for improving the stability of yttrium stabilised zirconia for dental restorations comprising yttrium stabilised zirconia, the dental restorations are veneered with a dental ceramic veneering material comprising the steps:
- providing a dental restoration comprising yttrium stabilised zirconia and to be veneered;
- applying the dental ceramic veneering material to the dental restoration by spraying-on an aerosol which contains the dental ceramic veneering material and water-free propellant;
- firing the resulting arrangement to a resulting veneered dental restoration, wherein
wherein the propellant is selected from the group consisting of aerosols based on heptafluoro propane or propane, Butane or mixtures thereof and
wherein the dental ceramic veneering material is applied in a thickness from 0.01 mm to 0.5 mm.

2. The process of claim 1 wherein the yttrium stabilised zirconia dental restoration comprises the composition:
| | |
|---|---|
| zirconium dioxide: | of from 93 to 97 % by weight |
| yttrium oxide: | of from 3 to 5 % by weight |
| hafnium oxide: | of from 0 to 3 % by weight |
| aluminium oxide: | of from 0 to 1 % by weight |
| silicon oxide: | of from 0 to 1 % by weight. |

3. The process of claim 1 or 2 wherein the dental ceramic veneering material comprises the composition:
| | |
|---|---|
| SiO₂: | of from 58 to 70 % by weight |
| Al₂O₃: | of from 10 to 18 % by weight |
| K₂O : | of from 6 to 12 % by weight |
| Na₂O: | of from 3 to 8 % by weight |
| B₂O₃: | of from 1 to 6 % by weight |
| BaO: | of from 0 to 3 % by weight |
| TiO₂: | of from 0 to 2 % by weight |
| CeO₂: | of from 0 to 2 % by weight |
| ZrO₂: | of from 0 to 2 % by weight |
| CaO: | of from 0 to 3 % by weight |
| SnO₂ : | of from 0 to 2 % by weight. |

4. The process of any one of the claims 1 to 3 wherein the dental ceramic veneering material is present in a particulate form, in particular of a powder having in particular a particle size of from 0.5 µm to 150 µm.

5. The process of any one of the claims 1 to 4 wherein the aerosol is released from a container by a propellant.

6. Use of a water-free aerosol for the manufacturing of a veneered dental restoration the aerosol comprising a water-free propellant and a dental ceramic veneering material for spraying-on a yttrium stabilised zirconia dental restoration to be veneered with the dental ceramic veneering material, wherein the water-free propellant is selected from the group consisting of aerosols based on heptafluoro propane or propane, Butane or mixtures thereof, wherein the dental ceramic veneering material is applied in a thickness from 0.01 mm to 0.5 mm.

## Patentansprüche

1. Verfahren zum Verbessern der Stabilität von yttriumstabilisiertem Zirconiumoxid für Zahnrestaurationen, die yttriumstabilisiertes Zirconiumoxid umfassen, wobei die Zahnrestaurationen mit einem Dentalkeramik-Veneermaterial verblendet sind, umfassend die Schritte:
- Bereitstellen einer Zahnrestauration, die yttriumstabilisiertes Zirconiumoxid umfasst und verblendet werden soll;
- Auftragen des Dentalkeramik-Veneermaterials auf die Zahnrestauration durch Aufsprühen eines Aerosols, das das Dentalkeramik-Veneermaterial und ein wasserfreies Treibmittel enthält;
- Brennen der resultierenden Anordnung zu einer resultierenden verblendeten Zahnrestauration, wobei
wobei das Treibmittel aus der Gruppe ausgewählt ist, die aus Aerosolen auf der Basis von Heptafluorpropan oder Propan, Butan oder Gemischen davon besteht; und
wobei das Dentalkeramik-Veneermaterial in einer Dicke von 0,01 mm bis 0,5 mm aufgetragen wird.

2. Verfahren gemäß Anspruch 1, wobei die yttriumstabilisiertes Zirconiumoxid umfassende Zahnrestauration die folgende Zusammensetzung umfasst:
| | |
|---|---|
| Zirconiumdioxid: | 93 bis 97 Gew.-% |
| Yttriumoxid : | 3 bis 5 Gew.-% |
| Hafniumoxid : | 0 bis 3 Gew.-% |
| Aluminiumoxid: | 0 bis 1 Gew.-% |
| Siliciumoxid : | 0 bis 1 Gew.-%. |

3. Verfahren gemäß Anspruch 1 oder 2, wobei das Dentalkeramik-Veneermaterial die folgende Zusammensetzung umfasst:
| | |
|---|---|
| SiO₂: | 58 bis 70 Gew.-% |
| Al₂O₃: | 10 bis 18 Gew.-% |
| K₂O: | 6 bis 12 Gew.-% |
| Na₂O: | 3 bis 8 Gew.-% |
| B₂O₃: | 1 bis 6 Gew.-% |
| BaO: | 0 bis 3 Gew.-% |
| TiO₂: | 0 bis 2 Gew.-% |
| CeO₂: | 0 bis 2 Gew.-% |
| ZrO₂: | 0 bis 2 Gew.-% |
| CaO: | 0 bis 3 Gew.-% |
| SnO₂: | 0 bis 2 Gew.-%. |

4. Verfahren gemäß einem der Ansprüche 1 bis 3, wobei das Dentalkeramik-Veneermaterial in Teilchenform vorliegt, insbesondere als Pulver, das insbesondere eine Teilchengröße von 0,5 µm bis 150 µm aufweist.

5. Verfahren gemäß einem der Ansprüche 1 bis 4, wobei das Aerosol durch ein Treibmittel aus einem Behälter freigesetzt wird.

6. Verwendung eines wasserfreien Aerosols zur Herstellung einer verblendeten Zahnrestauration, wobei das Aerosol ein wasserfreies Treibmittel und ein Dentalkeramik-Veneermaterial zum Aufsprühen auf eine yttriumstabilisiertes Zirconiumoxid umfassende Zahnrestauration, die mit dem Dentalkeramik-Veneermaterial verblendet werden soll, umfasst, wobei das wasserfreie Treibmittel aus der Gruppe ausgewählt ist, die aus Aerosolen auf der Basis von Heptafluorpropan oder Propan, Butan oder Gemischen davon besteht, wobei das Dentalkeramik-Veneermaterial in einer Dicke von 0,01 mm bis 0,5 mm aufgetragen wird.

## Revendications

1. Procédé pour améliorer la stabilité de zircone stabilisée par l'yttrium pour des restaurations dentaires comprenant du zircone stabilisée par l'yttrium, les restaurations dentaires étant plaquées avec un matériau de placage en céramique dentaire, comprenant les étapes consistant à :
- procurer une restauration dentaire comprenant du zircone stabilisée par l'yttrium, qui doit être plaquée,
- appliquer le matériau de placage en céramique dentaire à la restauration dentaire en projetant un aérosol contenant le matériau de placage en céramique dentaire et un gaz propulseur anhydre,
- cuire l'arrangement résultant pour obtenir une restauration dentaire plaquée résultante, dans lequel
dans lequel ledit gaz propulseur est choisi dans le groupe consistant en des aérosols basés sur heptafluoropropane ou propane, butane ou des mélanges de ceux-ci, et
dans lequel ledit matériau de placage en céramique dentaire est appliqué en une épaisseur allant de 0,01 mm à 0,5 mm.

2. Procédé selon la revendication 1, dans lequel ladite restauration dentaire comprenant du zircone stabilisée par l'yttrium comprend la composition suivante :
| | |
|---|---|
| zircone: | 93 à 97 % en poids |
| oxyde d'yttrium: | 3 à 5 % en poids |
| oxyde de hafnium: | 0 à 3 % en poids |
| alumine: | 0 à 1 % en poids |
| silice: | 0 à 1 % en poids. |

3. Procédé selon la revendication 1 ou 2, dans lequel ledit matériau de placage en céramique dentaire comprend la composition suivante :
| | |
|---|---|
| SiO₂: | 58 à 70 % en poids |
| Al₂O₃: | 10 à 18 % en poids |
| K₂O: | 6 à 12 % en poids |
| Na₂O: | 3 à 8 % en poids |
| B₂O₃: | 1 à 6 % en poids |
| BaO: | 0 à 3 % en poids |
| TiO₂: | 0 à 2 % en poids |
| CeO₂: | 0 à 2 % en poids |
| ZrO₂: | 0 à 2 % en poids |
| CaO: | 0 à 3 % en poids |
| SnO₂: | 0 à 2 % en poids. |

4. Procédé selon l'une quelconque des revendications 1 à 3, dans lequel ledit matériau de placage en céramique dentaire est présent sous forme parti-culaire, notamment d'une poudre ayant notamment une granulométrie de 0,5 µm à 150 µm.

5. Procédé selon l'une quelconque des revendications 1 à 4, dans lequel ledit aérosol est libéré d'un récipient au moyen d'un gaz propulseur.

6. Utilisation d'un aérosol anhydre pour préparer une restauration dentaire plaquée, dans laquelle ledit aérosol comprend un gaz propulseur anhydre et un matériau de placage en céramique dentaire pour être projeté sur une restauration dentaire comprenant du zircone stabilisée par l'yttrium qui doit être plaquée avec ledit matériau de placage en céramique dentaire, dans lequel ledit gaz propulseur est choisi dans le groupe consistant en des aérosols basés sur heptafluoropropane ou propane, butane ou des mélanges de ceux-ci, dans lequel ledit matériau de placage en céramique dentaire est appliqué en une épaisseur allant de 0,01 mm à 0,5 mm.
